# EUROPEAN PATENT APPLICATION

(11) **EP 2 554 682 A1**
(43) Date of publication of application: **06.02.2013**
(21) Application number: 12179172.7
(22) Date of filing: 03.08.2012
(51) Int. Cl.: C12Q 1/68

(54) **Methods for Treating Myelodysplastic Syndrome with Ezatiostat**

(30) Priority: 05.08.2011 US 201161515626 P; 02.04.2012 US 201213437474
(71) Applicant: Telik, Inc., Palo Alto, CA 94304 (US)
(72) Inventor: Brown, Gail L., Palo Alto, CA California 94304 (US)
(74) Representative: Gibson, Mark

(57) **Abstract**

This invention relates to methods, assays, devices and systems for identifying patients having a myelodysplastic syndrome for treatment with ezatiostat or a salt thereof, or evaluating the patient's response possibility to the treatment by measuring and evaluating the patient's gene expression profile. This invention also relates to methods of treating myelodysplastic syndromes.

## Description

### Field of the Invention

This invention relates to methods, assays, devices and systems for identifying patients with myelodysplastic syndrome for treatment with ezatiostat or a salt thereof and methods for treating patients identified as having a myelodysplastic syndrome with ezatiostat or a salt thereof.

### State of the Art

Myelodysplastic syndrome(s) (MDS) refers to a heterogeneous group of clonal hematopoietic stem cell disorders characterized by ineffective hematopoiesis (blood cell production) involving one or more cell lineages (red blood cells, white blood cells or platelets) and a variable risk of transformation to acute myeloid leukemia (AML). This syndrome becomes more common with age. It is estimated that MDS affects approximately 300,000 people worldwide. According to the American Cancer Society, 10,000 to 20,000 new cases of MDS are diagnosed each year in the United States alone. Survival rates using current therapy range from 6 months to 6 years with patients often requiring blood transfusions to manage their disease.

Ezatiostat and its salts are disclosed in US Patent No. 5,763,570. Ezatiostat has the IUPAC chemical name of ethyl (2*S*)-2-amino-5-[[(2*R*)-3-benzylsulfanyl-1-[[(1*R*)-2-ethoxy-2-oxo-1-phenylethyl]amino]-1-oxopropan-2-yl]amino]-5-oxopentanoate.

One example of a salt of ezatiostat is the hydrochloride salt, ezatiostat hydrochloride (USAN), which has the molecular weight of 566.1, the trademark of Telintra®, and the CAS registry number of 286942-97-0. U.S. Patent Application Publication 2011/0301088, filed March 4, 2011, describes ansolvate and polymorphs of ezatiostat hydrochloride, which is incorporated by reference in its entirety.

Ezatiostat is an inhibitor of glutathione S-transferase (GST), an enzyme that is over expressed in many cancers, and has been shown *in vitro* to stimulate growth and differentiation of hematopoietic progenitor cells and to induce apoptosis in leukemia cells. Ezatiostat inhibits GST P1-1 which in turn leads to de-repression of jun kinase and subsequent activation of c-Jun. Activation of c-Jun results in normal hematopoietic progenitor cell proliferation and differentiation, and apoptosis of leukemia cells. This drug has been evaluated for the treatment of MDS, for example, in a Phase I-IIa study using a liposomal formulation (US Patent No. 7,029,695), as reported by Raza et al. in Journal of Hematology & Oncology, 2:20 (published online on 13 May 2009); and in a Phase I study using a tablet formulation, as reported by Raza et al. in Blood, 113:6533-6540 (prepublished online on 27 April 2009), and in a single patient case report by Quddus et al. in Journal of Hematology & Oncology, 3:16 (published online on 23 April 2010). Also see, Raza A., et al., Phase 2 Randomized Multicenter Study of Extended Dosing Schedules of Oral Ezatiostat HCl (Telintra), a Glutathione Analog Prodrug GSTP1-1 Inhibitor, in Low to Intermediate-1 Risk Myelodysplastic Syndrome (MDS), Proceedings of the American Society of Hematology Annual Meeting, December 4-7, 2010, Orlando, FL, Abstract #2910; and Raza A., et al., Phase 2 Randomized Multicenter Study of Two Extended Dosing Schedules of Oral Ezatiostat in Low to Intermediate-1 Risk Myelodysplastic Syndrome, Cancer, (first published online on 1 September 2011).

The entire disclosures of each of the patents, patent applications, and publications referred to in this application are incorporated into this application by reference.

### Summary of the Invention

Studies show that ezatiostat hydrochloride had varying success in treating MDS. This invention is predicated on the surprising discovery of the gene expression characteristics of MDS patients who responded to treatment with ezatiostat hydrochloride. Correlation of the gene expression with treatment of MDS by ezatiostat is not known, routine, or conventional. It is particularly surprising because of the heterogeneity of the cell lineages that comprise the malignant clone. This invention is not intended to preempt genetic identification in treating diseases, but is predicated on the discovery of a subpopulation of MDS patients who will likely benefit from the treatment with the specific drug of ezatiostat.

Tables 1 and 2 provide the top 100 differentially expressed genes found between patients who responded to the treatment (responders) and patients who did not respond to the treatment (non-responders). Table 3 provides pathways whose genes were found to be differentially expressed between responders and non-responders. As used herein, a gene of Table 3 refers to a gene of any of the pathways in Table 3. Table 4 provides genes in the JNK/JUN pathway that were found to be differentially expressed between responders and non-responders. Tables 5 and 6 provide the top 100 differentially expressed genes found between responders and non-responders when samples of a smaller set of patients were tested.

Accordingly, in one aspect, the invention is directed to a method for treating a myelodysplastic syndrome in a patient comprising administering a therapeutically effective amount of ezatiostat or a salt thereof, wherein the patient has an under-expression of a gene selected from Table 1, 3, 4 or 5, and/or an over-expression of a gene selected from Table 2 or 6.

In some embodiments, the invention is directed to a method for treating a myelodysplastic syndrome in a patient with an efficacious amount of ezatiostat or a salt thereof, which method comprising assaying a sample from said patient for the presence and determining expression level of a gene selected from Tables 1-6, administering ezatiostat to the patient provided that an under-expression of a gene selected from Table 1, 3, 4 or 5, and/or an over-expression of a gene selected from Table 2 or 6 is detected in the sample of the patient.

In another aspect, this invention is directed to a method for treating a myelodysplastic syndrome in a patient, comprising assaying a sample from said patient which assaying measures the presence and/or the expression level of a gene selected from Tables 1-6, and administering a therapeutically effective amount of ezatiostat or a salt thereof to the patient if an under-expression of a gene selected from Table 1, 3, 4 or 5, and/or an over-expression of a gene selected from Table 2 or 6 is detected in the sample of the patient. In some embodiments the gene is miR-129 or miR-155. In some embodiments, the patient is administered a therapeutically effective amount of ezatiostat or a salt thereof if an under-expression of miR-129, and/or an over-expression of miR-155 is detected in the sample. In some embodiments, the patient is administered a therapeutically effective amount of ezatiostat or a salt thereof if an under-expression of one or more genes, such as all, of the c-Jun N-terminal kinase gene set is detected in the sample.

In some embodiments, the method comprises measuring the expression level of a gene in the patient, wherein the gene is miR-129 or miR-155, and administering ezatiostat or a salt thereof to the patient if an under-expression of miR-129, and/or over-expression of miR-155 is detected in the patient. In some embodiments, a patient is administered ezatiostat or a salt thereof if an under-expression of a gene of the c-Jun N-terminal kinase gene set is detected in the patient. In some embodiments, the patient is administered a therapeutically effective amount of ezatiostat or a salt thereof if an under-expression of one or more genes of the mTOR, JAK2 or JNK pathway is detected in the patient.

In another aspect, this invention is directed to an improvement for treating a patient population suffering from myelodysplastic syndrome for which ezatiostat or a salt thereof has unpredictable success in treating the myelodysplastic syndrome, wherein the improvement comprises:
a) obtaining a biological sample from a patient;
b) assaying the sample and obtaining data relating to the presence and/or expression of a gene selected from Tables 1-6;
c) correlating the data from b) above to determine if one or more genes selected from Tables 1, 3, 4 and 5 are under-expressed and/or if one or more genes selected from Tables 2 and 6 are over-expressed;
d) initiating treatment of the patient with a therapeutically effective amount of ezatiostat or a salt thereof if an under-expression of one or more genes of Tables 1, 3, 4 and 5 and/or and over-expression of one or more genes of Tables 2 and 6 is determined;
e) ascertaining the tolerance of the patient to the treatment with ezatiostat or a salt thereof; and
f) continuing treatment with ezatiostat or a salt thereof only if the patient has an acceptable tolerance level.

In some embodiments, one or more genes under-expressed or over-expressed are determined as described herein.

In another aspect, this invention is directed to a method for evaluating the response probability of a patient having a myelodysplastic syndrome to treatment with ezatiostat or a salt thereof, said method comprising obtaining a biological sample from the patient and/or testing a biological sample of the patient, and detecting the presence and/or measuring expression level of a gene selected from Tables 1-6. It will be understood by the person skilled in the art that the methods described herein require the step of obtaining a biological sample from a patient followed by *in vitro* testing of the sample. In some embodiments, detection of an under-expression of a gene selected from Table 1, 3, 4 or 5 is indicative that the patient is likely to respond to the treatment. In some embodiments, the gene is one or more genes of the c-Jun N-terminal kinase gene set. In some embodiments, detection of an under-expression of one or more genes of the c-Jun N-terminal kinase gene set is indicative that the patient is likely to respond to the treatment. In some embodiments, detection of an under-expression of one or more genes of the mTOR, JAK2 or JNK pathway is indicative that the patient is likely to respond to the treatment. In certain embodiments, detection of an over-expression of a gene selected from Table 2 or 6 is indicative that the patient is likely to respond to the treatment.

In some embodiments, the gene is miR-129 or miR-155. In some embodiments, detection of an under-expression of miR-129 is indicative that the patient is likely to respond to the treatment. In certain embodiments, detection of an over-expression of miR-155 is indicative that the patient is likely to respond to the treatment.

In another aspect, this invention is directed to a method for identifying a patient having a myelodysplastic syndrome for treatment with ezatiostat or a salt thereof, said method comprising obtaining a biological sample from the patient and/or testing a biological sample of the patient, and detecting the presence and/or measuring expression level of a gene selected from Tables 1 to 6, wherein the patient is identified for the treatment if an under-expression of a gene selected from Table 1, 3, 4 or 5 is detected, and/or an over-expression of a gene selected from Table 2 or 6 is detected. In some embodiments, the method comprises the *in vitro* testing of a biological sample obtained from the patient. In some embodiments, the gene is miR-129. In some embodiments, the gene is miR-155.

In some embodiments, the patient is identified for the treatment if an under-expression of a gene selected from Tables 1, 4 and 5, preferably Table 1 or 4, is detected. In an alternative embodiment, the patient is identified for treatment if an under-expression of a gene selected from Tables 1, 3 or 4 is detected. In some embodiments, the patient is identified for the treatment if an under-expression of miR-129 is detected. In some embodiments, the patient is identified for the treatment if an under-expression of a gene of the c-Jun N-terminal kinase gene set is detected.

In certain embodiments, the patient is identified for the treatment if an over-expression of a gene selected from Table 2 is detected. In certain embodiments, the patient is identified for the treatment if an over-expression of a gene selected from Table 6 is detected. In certain embodiments, the patient is identified for the treatment if an over-expression of miR-155 is detected.

In some embodiments, the biological sample is bone marrow.

**Table 1**

| | Gene Symbol |
|---|---|
| 1-1. | HS.539400 |
| 1-2. | RUNDC3B |
| 1-3. | C8ORF48 |
| 1-4. | HS.153034 |
| 1-5. | MIR129-2 |
| 1-6. | OR10N4 |
| 1-7. | LOC401629 |
| 1-8. | LOC641860 |
| 1-9. | LOC100133511 |
| 1-10. | LOC389730 |
| 1-11. | DAZ3 |
| 1-12. | FAM90A3 |
| 1-13. | LRRC8E |
| 1-14. | SLC2A8 |
| 1-15. | PAEP |
| 1-16. | HS.368690 |
| 1-17. | HS.565863 |
| 1-18. | KLHDC9 |
| 1-19. | LOC652551 |
| 1-20. | PIGV |
| 1-21. | TLX1NB |
| 1-22. | GPC6 |
| 1-23. | HS.545893 |
| 1-24. | SNX22 |
| 1-25. | HS.569271 |
| 1-26. | LOC654161 |
| 1-27. | MMP 19 |
| 1-28. | ARPM1 |
| 1-29. | CTGF |
| 1-30. | HS.408168 |
| 1-31. | HS.437395 |
| 1-32. | HS.515967 |
| 1-33. | LOC100133600 |
| 1-34. | LOC645718 |
| 1-35. | LOC728927 |
| 1-36. | NRIP3 |
| 1-37. | PSMD8 |
| 1-38. | TRIM73 |
| 1-39. | ZBTB7A |
| 1-40. | C10ORF116 |
| 1-41. | DEFB127 |
| 1-42. | HS.126108 |
| 1-43. | HS.188979 |
| 1-44. | HS.543520 |
| 1-45. | HS.553161 |
| 1-46. | KLC1 |
| 1-47. | KRT36 |
| 1-48. | LOC647928 |
| 1-49. | PABPC5 |
| 1-50. | LOC390530 |

**Table 2**

| | Gene Symbol |
|---|---|
| 2-1. | MUC19 |
| 2-2. | LOC643345 |
| 2-3. | FRMD3 |
| 2-4. | LOC644280 |
| 2-5. | C15ORF5 |
| 2-6. | LOC442245 |
| 2-7. | MIR155HG |
| 2-8. | IFNE1 |
| 2-9. | LOC647012 |
| 2-10. | LOC649908 |
| 2-11. | ROPN1 |
| 2-12. | AMPD2 |
| 2-13. | CNDP1 |
| 2-14. | ROCK1 |
| 2-15. | ANP32E |
| 2-16. | GAPT |
| 2-17. | SKIL |
| 2-18. | CASC1 |
| 2-19. | DDO |
| 2-20. | LOC100133435 |
| 2-21. | CLEC12A |
| 2-22. | FLJ41423 |
| 2-23. | GK5 |
| 2-24. | LOC100131542 |
| 2-25. | LOC641990 |
| 2-26. | ZNF791 |
| 2-27. | EFHC2 |
| 2-28. | LOC730909 |
| 2-29. | PDCD6IP |
| 2-30. | ZNF193 |
| 2-31. | C7ORF46 |
| 2-32. | CLDN12 |
| 2-33. | LOC100129387 |
| 2-34. | LOC100132955 |
| 2-35. | LOC647911 |
| 2-36. | LOC730173 |
| 2-37. | NME2P1 |
| 2-38. | NRSN1 |
| 2-39. | PRKCB |
| 2-40. | SNAP25 |
| 2-41. | ABCA2 |
| 2-42. | CDC2L5 |
| 2-43. | HS.463736 |
| 2-44. | HS.543956 |
| 2-45. | HS.545655 |
| 2-46. | KLK6 |
| 2-47. | LOC100132516 |
| 2-48. | LOC100133719 |
| 2-49. | LOC643717 |
| 2-50. | LOC728692 |

**Table 3**

| | |
|---|---|
| 3-1. | H2O2_CSBRSCUED_C2_UP |
| 3-2. | AGED_MOUSE_HIPPOCAMPUS_ANY |
| 3-3. | mTOR_UP.n4.v1 |
| 3-4. | JAK2_DN.v1 |
| 3-5. | CAMPTOTHECIN PROBCELL |
| 3-6. | HDACI COLON BUT |
| 3-7. | LEF1_UP.v1 |
| 3-8. | MATRIX_METALLOPROTEINASES |
| 3-9. | OXIDATIVE_PHOSPHORYLATION |
| 3-10. | HYPOPHYSECTOMY_RAT |
| 3-11. | NITROGEN_METABOLISM |
| 3-12. | ZHAN_MM_CD138_CD1_VS_REST |
| 3-13. | CAMPTOTHECIN_PROBCELL_UP |
| 3-14. | EGFR_II_UP.v1 |
| 3-15. | NI2_LUNG_DN |
| 3-16. | Cyclin_D1_UP.v1 |
| 3-17. | SLRPPATHWAY |
| 3-18. | HSA00271_METHIONINE_METABOLISM |
| 3-19. | HSA00910_NITROGEN_METABOLISM |
| 3-20. | ALK.DN.v1 |
| 3-21. | ALK_DN.v_1_UP |
| 3-22. | EGFR_II_UP.v1_UP |
| 3-23. | INNEREAR_UP |
| 3-24. | O6BG_RESIST_MEDULLOBLASTOMA |
| 3-25. | HSA05130_PATHOGENIC_ESCHERICHIACOLI_INFECTION_EHEC |
| 3-26. | HSA05131_PATHOGENIC_ESCHERICHIA_COLI_INFECTION_EHEC |
| 3-27. | HOXA9_UP.v1 |
| 3-28. | JNK_UP.v1 |
| 3-29. | METHIONINE_METABOLISM |
| 3-30. | MARCINIAK_CHOP_DIFF |
| 3-31. | HDACI_COLON_CUR48HRS_UP |
| 3-32. | NUTT_GBN_VS_AO_DN |
| 3-33. | MTA3PATHWAY |
| 3-34. | COMPLEMENT_ACTIVATION_CLASSICAL |
| 3-35. | BROWN_MYELOID_PROLIF_AND_SELF_RENEWAL |
| 3-36. | TSA_HEPATOMA_CANCER_UP |
| 3-37. | HDACI_COLON_TSA2HRS |
| 3-38. | ALZHEIMERS_DISEASE |
| 3-39. | CALRES_MOUSE |
| 3-40. | HBX_HEP_DN |
| 3-41. | CMV_HCMV_TIMECOURSE_10HRS |
| 3-42. | STRESS_GENOTOXIC_SPECIFIC_UP |
| 3-43. | HDACI COLON SUL2HRS UP |
| 3-44. | O6BG_RESIST_MEDULLOBLASTOMA_UP |
| 3-45. | HSA0040_NOVOBIOCIN_BIOSYNTHESIS |
| 3-46. | Cyclin_D1_UP.v1_UP |
| 3-47. | KENNY_WNT |
| 3-48. | METHIONINEPATHWAY |
| 3-49. | UREACYCLEPATHWAY |
| 3-50. | HDACI_COLON_CURSUL_UP |

**Table 4**

| | Gene Symbol |
|---|---|
| 4-1. | C1ORF144 |
| 4-2. | IFNA2 |
| 4-3. | KIF22 |
| 4-4. | ANKRD1 |
| 4-5. | FLRT1 |
| 4-6. | ANXA13 |
| 4-7. | PATHWAY:JNK_UP.v1 |
| 4-8. | PLAA |
| 4-9. | BEAN |
| 4-10. | SOX10 |
| 4-11. | EIF2C2 |
| 4-12. | CYP1A1 |
| 4-13. | PLK1 |
| 4-14. | KRT75 |
| 4-15. | CCL15 |
| 4-16. | ARL4A |
| 4-17. | C10ORF10 |
| 4-18. | TEK |
| 4-19. | FABP6 |
| 4-20. | TULP1 |
| 4-21. | ABHD2 |
| 4-22. | C1S |
| 4-23. | UBE2C |
| 4-24. | PDGFB |
| 4-25. | DYRK3 |
| 4-26. | RAB6B |
| 4-27. | JPH3 |
| 4-28. | SYT17 |
| 4-29. | G0S2 |
| 4-30. | PIB5PA |
| 4-31. | HIST1H2AM |
| 4-32. | KCNN3 |
| 4-33. | HIST1H2AK |
| 4-34. | MPL |
| 4-35. | ASGR1 |
| 4-36. | SHC3 |
| 4-37. | RRAD |
| 4-38. | MRAS |
| 4-39. | CXCL11 |
| 4-40. | ABP1 |
| 4-41. | ALOX12B |
| 4-42. | IL8 |
| 4-43. | Fll |
| 4-44. | PCDH9 |
| 4-45. | MMP2 |
| 4-46. | NFKBIL1 |
| 4-47. | CDKN3 |
| 4-48. | TACR1 |
| 4-49. | ECGF1 |
| 4-50. | GJA4 |

**Table 5**

| | Gene Symbol |
|---|---|
| 5-1. | LOC642539 |
| 5-2. | TNN |
| 5-3. | LOC651630 |
| 5-4. | OR6C6 |
| 5-5. | FAM134A |
| 5-6. | HS.543561 |
| 5-7. | HS.539400 |
| 5-8. | C17ORF102 |
| 5-9. | CHAD |
| 5-10. | HS.156574 |
| 5-11. | LOC642228 |
| 5-12. | LOC650407 |
| 5-13. | FAM90A5 |
| 5-14. | LOC648408 |
| 5-15. | RUNDC3B |
| 5-16. | CDRT15L2 |
| 5-17. | LOC653579 |
| 5-18. | LOC645781 |
| 5-19. | CD300E |
| 5-20. | CFL2 |
| 5-21. | C8ORF48 |
| 5-22. | LOC651166 |
| 5-23. | HS.153034 |
| 5-24. | HCRT |
| 5-25. | SLC22A9 |
| 5-26. | DPYSL5 |
| 5-27. | LOC646799 |
| 5-28. | LOC649125 |
| 5-29. | LOC644059 |
| 5-30. | MIR129-2 |
| 5-31. | OR10A4 |
| 5-32. | LOC653536 |
| 5-33. | LOC728255 |
| 5-34. | SNORD72 |
| 5-35. | MIR802 |
| 5-36. | LOC645839 |
| 5-37. | HS.574590 |
| 5-38. | CPSF1 |
| 5-39. | LOC401629 |
| 5-40. | FLJ35894 |
| 5-41. | MIR548E |
| 5-42. | MUC4 |
| 5-43. | LOC649878 |
| 5-44. | LOC642214 |
| 5-45. | OTOL1 |
| 5-46. | LOC641860 |
| 5-47. | SCNN1G |
| 5-48. | LOC340204 |
| 5-49. | LOC642561 |
| 5-50. | LOC100133511 |

**Table 6**

| | Gene Symbol |
|---|---|
| 6-1. | MUC19 |
| 6-2. | GLB1L |
| 6-3. | LOC100129503 |
| 6-4. | FGF22 |
| 6-5. | SRR |
| 6-6. | IPMK |
| 6-7. | LACE1 |
| 6-8. | EIF1AD |
| 6-9. | FAM114A1 |
| 6-10. | LOC100130345 |
| 6-11. | CLCF1 |
| 6-12. | HS.149786 |
| 6-13. | LOC100133639 |
| 6-14. | KIAA1107 |
| 6-15. | CNP |
| 6-16. | LOC643345 |
| 6-17. | HS.550095 |
| 6-18. | ANKMY2 |
| 6-19. | CCNI2 |
| 6-20. | LOC730517 |
| 6-21. | STRA8 |
| 6-22. | GPX7 |
| 6-23. | FRMD3 |
| 6-24. | LOC644280 |
| 6-25. | TMX3 |
| 6-26. | C15ORF5 |
| 6-27. | KHDC1 |
| 6-28. | LOC100128088 |
| 6-29. | LOC643402 |
| 6-30. | LOC100132169 |
| 6-31. | MRS2P2 |
| 6-32. | BRCC3 |
| 6-33. | LOC729242 |
| 6-34. | RBMY3AP |
| 6-35. | OR3A3 |
| 6-36. | ARL17B |
| 6-37. | LOC440330 |
| 6-38. | FLJ46109 |
| 6-39. | PARP8 |
| 6-40. | LOC100130982 |
| 6-41. | FAM83E |
| 6-42. | LOC442245 |
| 6-43. | SYT14 |
| 6-44. | HS.570965 |
| 6-45. | TSPAN32 |
| 6-46. | IFNE1 |
| 6-47. | C14ORF148 |
| 6-48. | SLC16A14 |
| 6-49. | LOC728297 |
| 6-50. | miR-155 |

Another aspect of the invention is directed to use of a gene selected from Tables 1-6 for identifying a patient having a myelodysplastic syndrome for treatment with ezatiostat or a salt thereof, said use comprising obtaining a biological sample from the patient, and testing the biological sample to detect the presence and/or measure expression level of a gene selected from Tables 1-6. The patient is identified for treatment if an under-expression of a gene selected from Tables 1 and 3-5, and/or an over-expression of a gene selected from Table 2 or 6 is detected.

Another aspect of the invention is directed to use of a gene selected from Tables 1-6 for evaluating the response probability of a patient having a myelodysplastic syndrome to treatment with ezatiostat or a salt thereof, said use comprising obtaining a biological sample from the patient, and testing the biological sample to detect the presence and/or measure expression level of the gene. Detection of an under-expression of a gene selected from Tables 1 and 3-5, and/or an over-expression of a gene selected from Table 2 or 6 is indicative that the patient is likely to respond to the treatment.

Another aspect of the invention is directed to use of a gene selected from Tables 1-6 for treating a myelodysplastic syndrome in a patient, comprising measuring the expression level of the gene in the patient, wherein the patient is administered a therapeutically effective amount of ezatiostat or a salt thereof if an under-expression of a gene selected from Tables 1 and 3-5, and/or an over-expression of a gene selected from Table 2 or 6 is detected in the patient.

Yet another aspect of the invention is an assay for identifying a patient having a myelodysplastic syndrome to treatment with ezatiostat or a salt thereof, and/or for evaluating the response probability of a patient having a myelodysplastic syndrome to treatment with ezatiostat or a salt thereof, the assay comprising testing a biological sample of the patient, and detecting the presence and/or measuring expression level of a gene selected from Tables 1-6 in the sample. The patient is identified for treatment or considered likely to respond to the treatment if an under-expression of a gene selected from Tables 1 and 3-5, and/or an over-expression of a gene selected from Table 2 or 6 is detected.

Yet another aspect of the invention is an assay for identifying a patient suffering from a myelodysplastic syndrome for whom there is an enhanced response rate to treatment with ezatiostat or a salt thereof, which the assay comprises
testing a biological sample of the patient, and detecting the presence and/or measuring expression level of a gene selected from Tables 1-6 in the sample,
determining the expression level of said gene, ascertaining the presence of an under-expression of one or more genes selected from Tables 1, 3, 4 and 5, and/or an over-expression of one or more genes selected from Tables 2 and 6,
correlating the patient's response rate to the treatment wherein the detection of either an under-expression of one or more genes selected from Tables 1, 3, 4 and 5, and/or an over-expression of one or more genes selected from Tables 2 and 6, indicates that the patient has an enhanced response rate to treatment with ezatiostat or a salt thereof.

Yet another aspect of the invention is an assay for identifying a patient suffering from a myelodysplastic syndrome for whom there is an enhanced probability of response to treatment with ezatiostat or a salt thereof, wherein the assay comprises
detecting the presence and/or measuring expression level of a gene selected from Tables 1-6 in the sample,
identifying the expression level of said genes,
ascertaining the presence of an under-expression of one or more genes selected from Tables 1, 3, 4 and 5, and/or an over-expression of one or more genes selected from Tables 2 and 6,
correlating the presence of an under-expression of one or more genes selected from Tables 1, 3, 4 and 5, and/or an over-expression of one or more genes selected from Tables 2 and 6 with the patient's probability of response to the treatment wherein the detection of either an under-expression of one or more genes selected from Tables 1, 3, 4 and 5, or an over-expression of one or more genes selected from Tables 2 and 6, indicates that the patient has an enhanced likelihood of response to treatment with ezatiostat or a salt thereof.

Yet another aspect of the invention is an assay for assaying the response rate of a patient suffering a myelodysplastic syndrome to treatment with ezatiostat or a salt thereof, and/or for evaluating the response probability of a patient having a myelodysplastic syndrome to treatment with ezatiostat or a salt thereof, the assay comprising means for the detection of an under-expression of a gene selected from Tables 1 and 3-5, and/or an over-expression of a gene selected from Table 2 or 6.

Yet another aspect of the invention is a device for identifying a patient suffering from a myelodysplastic syndrome for whom there is an enhanced probability of response to treatment with ezatiostat or a salt thereof, said device comprising an output medium that indicates whether there is an under-expression of one or more genes selected from Tables 1, 3, 4 and 5, and/or an over-expression of one or more genes selected from Tables 2 and 6 in the patient.

Yet another aspect of the invention provides a non-transitory computer-readable medium comprising code which when executed determines whether an under-expression of one or more genes selected from Tables 1, 3, 4 and 5, and/or an over-expression of one or more genes selected from Tables 2 and 6 is present in a sample obtained from a patient having myelodysplastic syndrome. In some embodiments, the code further determines the patient's probability of response to treatment with ezatiostat or a salt thereof and/or identifies the patient for treatment with ezatiostat or a salt thereof.

Yet another aspect of the invention provides a computer system comprising a processor, a memory, and code which when executed determines whether an under-expression of one or more genes selected from Tables 1, 3, 4 and 5, and/or an over-expression of one or more genes selected from Tables 2 and 6 is present in a sample obtained from a patient having myelodysplastic syndrome. In some embodiments, the system further determines the patient's probability of response to treatment with ezatiostat or a salt thereof and/or identifies the patient for treatment with ezatiostat or a salt thereof.

In some embodiments, ezatiostat or a salt thereof is administered by a dosing regimen described in U.S. Patent Application Publication US2011/0301102, titled "COMPOSITIONS AND METHODS FOR TREATING MYELODYSPLASTIC SYNDROME," filed May 16, 2011, which is incorporated by reference in its entirety. For example, ezatiostat hydrochloride may be administered in cycles of 2 gram/day orally for 3 weeks on/1 week off, or cycles of 3 gram/day orally for 2 weeks on/1 week off. Equivalent ezatiostat doses of ezatiostat itself or other ezatiostat salts, or other routes of administration may also be used.

In one embodiment, ezatiostat or a salt thereof can be administered as a tablet formulation. Such a tablet formulation is disclosed in U.S. Patent Application Publication US2011/0300215, filed March 29, 2011, titled "TABLET FORMULATION OF EZATIOSTAT," which is incorporated by reference in its entirety.

These and other embodiments of this invention are further described in the text that follows.

### Detailed Description of the Invention

Prior to describing this invention in greater detail, the following terms will first be defined.

It is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

It must be noted that as used herein and in the appended claims, the singular forms "a", "an", and "the" include plural referents unless the context clearly dictates otherwise. Thus, for example, reference to "a gene" includes a plurality of genes.

### 1. Definitions

Unless defined otherwise, all technical and scientific terms used herein have the same meaning as commonly understood by one of ordinary skill in the art to which this invention belongs. As used herein the following terms have the following meanings.

The term "comprising" or "comprises" means that the compositions and methods include the recited elements, but do not exclude others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" means excluding more than trace elements of other ingredients and substantial method steps. Embodiments defined by each of these transition terms are within the scope of this invention.

The term "about" when used before a numerical designation, e.g., temperature, time, amount, and concentration, including range, indicates approximations which may vary by (+) or (-) 15%, 10%, 5% or 1%.

As used herein, "expression" refers to the process by which polynucleotides are transcribed into mRNA and/or the process by which the transcribed mRNA is subsequently translated into peptides, polypeptides or proteins. If the polynucleotide is derived from genomic DNA, expression may include splicing of the mRNA in an eukaryotic cell.

"Differentially expressed" as applied to a gene, refers to the differential production of the mRNA transcribed and/or translated from the gene or the protein product encoded by the gene. A differentially expressed gene may be over-expressed or under-expressed as compared to the expression level of a normal or control cell, or other standards, such as that of subjects having a different gene profile or historical data. The term "differentially expressed" also refers to nucleotide sequences in a cell or tissue which are expressed where silent in a control cell or not expressed where expressed in a control cell.

As used herein, "over-expression" generally is at least 1.25 fold or, alternatively, at least 1.5 fold or, alternatively, at least 2 fold expression, or alternatively, at least 4 fold expression over that detected in a normal or healthy counterpart cell or tissue, or another standard such as that of subjects having a different gene profile or historical data. In some embodiments, "over-expressed" refers to an expression level that is higher than the expression level of a patient who does not respond to the treatment. A high expression level of the gene may occur because of over expression of the gene or an increase in gene copy number. The gene may also be translated into more protein because of deregulation of a negative regulator.

As used herein, "under-expression" generally is at least 5 % or, alternatively, at least 10 % or, alternatively, at least 20 %, or alternatively, at least 50 % less than the expression detected in a normal or healthy counterpart cell or tissue, or another standard such as that of subjects having a different gene profile or historical data. In some embodiments, "under-expressed" refers to an expression level that is lower than the expression level of a patient who does not respond to the treatment.

As used herein, "gene expression profile" refers to a pattern of expression of a set of genes that recurs in multiple samples and reflects a property shared by those samples, such as tissue type, response to a particular treatment, or activation of a particular biological process or pathway in the cells. A gene expression profile may be used to predict whether samples of unknown status share that common property or not. For example, it may be used to diagnose whether a patient has a certain illness or screen for patients within a category, such as those most likely to respond to a certain type of treatment. Some variation between the levels of the individual genes of the set and the typical profile is to be expected, but the overall similarity of the expression levels to the typical profile is such that it is statistically unlikely that the similarity would be observed by chance in samples not sharing the common property that the expression profile reflects.

The term "therapeutically effective amount" refers to the amount of ezatiostat or a salt thereof that is an amount sufficient to effect treatment, as defined herein, when administered to a subject in need of such treatment. In one embodiment, the therapeutically effective amount will be up to 3.5 grams (g) of ezatiostat or a salt thereof administered per day. Preferably, ezatiostat or a salt thereof is administered in an amount of 2 grams per day and, more preferably, is administered twice a day in equal 1 gram doses. Such a therapeutically effective amount is particularly relevant when the treatment regimen is for 3 weeks of administration of ezatiostat or a salt thereof followed by a week of no administration of the drug. In another embodiment, the therapeutically effective amount will be up to 3 grams of ezatiostat or a salt thereof administered in a single dose, or in 2 equal daily doses of up to 1.5 grams. Such a therapeutically effective amount is particularly relevant when the treatment regimen is for 2 weeks of administration of ezatiostat or a salt thereof followed by a week of no administration of the drug. Preferably, the dosing regimen employs 2 grams of ezatiostat or a salt thereof administered in an amount of 1 gram doses twice a day either under continuous administration or with administration for 3 weeks followed by a week of no administration of the drug.

As used herein, the term "treatment" or "treating" means any treatment of MDS in a patient which produces one or more of the following:
- inhibiting the MDS, that is, arresting or suppressing the development of symptoms (e.g., need for blood transfusion, abnormal blood count, and the like); and/or
- relieving the MDS, that is, causing the regression of symptoms.

As used herein, the term "response to treatment" means that a patient's MDS is inhibited or relieved after being given the treatment. The response can be temporary or permanent.

As used herein, the term "enhanced probability of response to treatment" or "enhanced response rate" means that the probability or rate of a particular MDS patient to respond to a particular treatment is higher than that of the general MDS patient population. The term "likely to response to treatment" means that the patient is more likely to respond to the treatment than not to respond to the treatment.

As used herein, the term "tolerance of the patient to the treatment" refers to the ability of a patient to tolerate the level of side effects caused by ezatiostat, such as nausea, diarrhea, vomiting, abdominal pain, constipation, anorexia and dyspepsia.

As used herein, the term "acceptable tolerance level" means that the side effects of ezatiostat is not severe enough to cause withdrawal of the treatment. This includes situations where the side effects are minimal or tolerable to a patient, or where the side effects of ezatiostat do not outweigh the benefit of the treatment as determined by an attending physician.

As used herein, the term "patient" refers to mammals and includes humans and non-human mammals. In some embodiments, the patient is a human patient.

As used herein, the term "output medium" refers to any medium that can be used to store, display and/or retrieve data or results of a determination of the presence and/or expression level of one or more genes. Such output medium includes, but is not limited to, a computer screen, memory, flash drive, compact disk, printed documents, etc. In some embodiments, the output medium is in a retrievable form. Retrievability of the data is important for keeping records of patient's gene profile, treatment history, determination of further treatment, correlating patient's actual response with the gene profile, and provides enhanced information regarding the gene profiles of MDS patients who are suitable for treatment with ezatiostat for potentially improved identification of patients for treatment with ezatiostat and prediction of patients' response to the treatment, etc. The data or results can be stored and/or display as raw data obtained from gene analysis, in digital form or as plots, images, or can be stored and/or display after being processed by a computer to various degrees. For example, the data can be processed by normalization and/or rescaling, or can be processed to display a conclusion as to whether and which genes are under- or over-expressed, or can be processed to display a patient's probability of response to treatment. Examples of data/results storage and/or display form include clustering images, heat maps, principal component analysis plots, or any chart indicating classification of samples (e.g., responder or non-responder), etc, which are known in the art.

As used herein, the term "computer-readable medium" refers to a device for recording information. Examples of computer-readable medium are known in the art. In one embodiment, a computer-readable medium is a computer hard drive. In another embodiment, a computer-readable medium is a computer memory. In another embodiment, a computer-readable medium is a flash drive, compact disk, or other portable devices. In yet another embodiment, a computer-readable medium is provided by a cloud server.

### 2. Methods

In one aspect, this invention is directed to a method for identifying a patient having myelodysplastic syndrome for treatment with ezatiostat or a salt thereof, said method comprising obtaining a biological sample from the patient, and detecting the presence and/or measuring expression level of a gene selected from Tables 1-6 in the sample. In some embodiments, the presence and/or expression level of one or multiple genes selected from Table 1 and/or Table 2, and the genes of the c-Jun N-terminal kinase gene set is measured. In some embodiments, the patient is identified for the treatment if an under-expression of a gene selected from Table 1, and 3-5 is detected. In certain embodiments, the patient is identified for the treatment if an over-expression of a gene selected from Table 2 or 6 is detected. In some embodiments, the patient is identified for the treatment if an under-expression of one or more genes of a pathway selected from those in Table 3 is detected. In some embodiments, the pathway is the mTOR, JAK2 (tyrosine Janus Kinase-2) or JNK pathway. mTOR relates to the serine/threonine kinase Akt, PI3K (phosphoinositide-3 kinase), receptor tyrosine kinases (RTKs), including epidermal growth factor receptor (EGFR), insulin-like growth factor-1 receptor (IGF-1R), and G protein-coupled receptors (GPCRs). In some embodiments, the pathway is the JNK/JUN pathway. In some embodiments, the patient is identified for the treatment if an under-expression of a gene selected from Table 4 is detected. In some embodiments, the patient is identified for the treatment if the patient has a gene-set profile of the JNK-inhibited keratinocytes.

In some embodiments, the gene is miR-129 and/or miR-155. In some embodiments, the patient is identified for the treatment if an under-expression of miR-129 is detected. In some embodiments, the patient is identified for the treatment if an under-expression of all miR-129, miR-802 and miR-548e is detected. In certain embodiments, the patient is identified for the treatment if an over-expression of miR-155 is detected. In some embodiments the under-expressed gene is miR-129 or the over-expressed gene is miR-155. In some embodiments, the patient is selected for treatment if an under-expression of one or more or all of OR10A4, RUNDC3B, C8ORF48, HS.539400, LOC100133511, HS.153034, miR-129, LOC401629, and LOC641860, and/or an over-expression of one or more or all of LOC442245, FRMD3, IFNE1, MUC19, LOC643345, C15ORF5, LOC644280, and miR-155 is detected in the patient.

In some embodiments, the patient is identified for the treatment if an under-expression of a gene of the jun-N-terminal kinase/c-Jun molecular pathway is detected. In some embodiments, the patient is identified for the treatment if an under-expression of a gene selected from Table 3 or 4 is detected. In some embodiments, the patient is identified for the treatment if an under-expression of one or more genes of the c-Jun N-terminal kinase gene set is detected. In some embodiments, the patient is identified for the treatment if an under-expression of all of the c-Jun N-terminal kinase genes is detected.

In some embodiments, the patient is identified for the treatment if an under-expression of at least 5, 10, 15, 20, 25, 30, 35, 40 or 45 of the genes of any one of Tables 1 and 3-5 or the genes of the c-Jun N-terminal kinase genes is detected. In some embodiments, the patient is identified for the treatment if an over-expression of at least 5, 10, 15, 20, 25, 30, 35, 40 or 45 of the genes of Table 2 or 6 is detected. In some embodiments, a patient is identified for the treatment, if an under-expression of multiple genes, for example, at least 10 %, 20 %, 50 % or 75 % of the genes of Tables 1, 3, 4, and/or 5, and/or the c-Jun N-terminal kinase gene set, and/or an over-expression of multiple genes selected from Table 2 and/or 6, for example, at least 10 %, 20 %, 50 % or 75 % of the genes of Tables 2 and/or 6, is observed.

In another aspect, this invention is directed to a method for evaluating the response probability of a patient having a myelodysplastic syndrome to treatment with ezatiostat or a salt thereof, said method comprising obtaining a biological sample from the patient, and detecting the presence and/or measuring expression level of a gene selected from Tables 1-6 in the sample. In some embodiments, the presence and/or expression level of one or multiple genes selected from Table 1 and/or Table 2, and the c-Jun N-terminal kinase gene set is measured. In some embodiments, detection of an under-expression of a gene selected from Table 1 and 3-5 is indicative that the patient is likely to respond to the treatment. In certain embodiments, detection of an over-expression of a gene selected from Table 2 or 6 is indicative that the patient is likely to respond to the treatment.

In some embodiments, detection of an under-expression of a gene of a pathway selected from those in Table 3 is indicative that the patient is likely to respond to the treatment. In some embodiments, the pathway is the mTOR, JAK2 or JNK pathway. In some embodiments, the pathway is the JNK/JUN pathway. In some embodiments, detection of an under-expression of a gene selected from Table 4 is indicative that the patient is likely to respond to the treatment. In some embodiments, the patient is likely to respond to the treatment if the patient has a gene-set profile of the JNK-inhibited keratinocytes.

In some embodiments, detection of an under-expression of one or more or all of OR10A4, RUNDC3B, C8ORF48, HS.539400, LOC100133511, HS.153034, miR-129, LOC401629, and LOC641860, and/or an over-expression of one or more or all of LOC442245, FRMD3, IFNE1, MUC19, LOC643345, C15ORF5, LOC644280, and miR-155 is indicative that the patient is likely to respond to the treatment.

In some embodiments, the gene is selected from the group consisting of miR-129, miR-802, miR-548e and miR-155. In some embodiments, detection of an under-expression of a gene selected from the group consisting of miR-129, miR-802, and miR-548e is indicative that the patient is likely to respond to the treatment. In some embodiments, detection of an under-expression of miR-129 is indicative that the patient is likely to respond to the treatment. In certain embodiments, detection of an over-expression of miR-155 is indicative that the patient is likely to respond to the treatment. In some embodiments, the expression of miR-129 and miR-155 is measured.

In some embodiments, detection of an under-expression of a gene of the c-Jun N-terminal kinase gene set is indicative that the patient is likely to respond to the treatment. In some embodiments, detection of an under-expression of all of the c-Jun N-terminal kinase genes is indicative that the patient is likely to respond to the treatment.

In some embodiments, detection of an under-expression of at least 5, 10, 15, 20, 25, 30, 35, 40 or 45 of the genes of any one of Tables 1 and 3-5 or the genes of the c-Jun N-terminal kinase genes is indicative that the patient is likely to respond to the treatment. In some embodiments, detection of an over-expression of at least 5, 10, 15, 20, 25, 30, 35, 40 or 45 of the genes of Table 2 or 6 is indicative that the patient is likely to respond to the treatment. In some embodiments, detection of an under-expression of multiple genes, for example, at least 10 %, 20 %, 50 % or 75 % of the genes of Table 1, 3, 4, and/or 5, and/or the c-Jun N-terminal kinase gene set, and/or an over-expression of multiple genes selected from Table 2 and/or 6, for example, at least 10 %, 20 %, 50 % or 75 % of the genes of Table 2 and/or 6 is indicative that the patient is likely to respond to the treatment.

Another aspect of the invention is a method for treating a myelodysplastic syndrome in a patient comprising administering a therapeutically effective amount of ezatiostat or a salt thereof, wherein the patient is detected to have an under-expression of a gene selected from Table 1, 3, 4 or 5, and/or an over-expression of a gene selected from Table 2 or 6. In some embodiments, the patient is detected to have one or more genes under-expressed or over-expressed as described herein.

Another aspect of the invention is a method for treating a myelodysplastic syndrome in a patient, comprising measuring the expression level of a gene in the patient by testing a biological sample of the patient, wherein the gene is selected from Tables 1-6, and administering a therapeutically effective amount of ezatiostat or a salt thereof to the patient if an under-expression of a gene selected from Table 1, 3, 4, or 5, and/or over-expression of a gene selected from Table 2 or 6 is detected in the patient.

In some embodiments, the expression of more than one gene is measured.

In some embodiments, the method comprises measuring the expression level of a gene in the patient, wherein the gene is selected from the group consisting of miR-129, miR-802, miR-548e and miR-155, and administering a therapeutically effective amount of ezatiostat or a salt thereof to the patient if an under-expression of a gene selected from miR-129, miR-802 and miR-548e, and/or over-expression of miR-155 is detected in the patient. In some embodiments, the expression of miR-129 and/or miR-155 is measured. In some embodiments, the patient is administered a therapeutically effective amount of ezatiostat or a salt thereof if an under-expression of miR-129 and/or an over-expression of miR-155 is detected in the patient.

In some embodiments, the method comprises measuring the expression level of a gene selected from Table 1, 2 or 4 in the patient by testing a sample obtained from the patient, and administering ezatiostat or a salt thereof to the patient if an under-expression of a gene selected from Table 1 or 4, and/or an over-expression of a gene selected from Table 2 is detected in the patient. In some embodiments, the method comprises measuring the expression level of a gene in a pathway selected from Table 3 in the patient, and administering ezatiostat or a salt thereof to the patient if an under-expression of the gene is detected in the patient. In some embodiments, the pathway is the mTOR, JAK2 or JNK pathway. In some embodiments, the pathway is the JNK/JUN pathway.

In some embodiments, the patient is administered a therapeutically effective amount of ezatiostat or a salt thereof if an under-expression of miR-129 and an over-expression of miR-155 is detected in the patient. In some embodiments, the patient is administered a therapeutically effective amount of ezatiostat or a salt thereof if an under-expression of one or more or all of OR10A4, RUNDC3B, C8ORF48, HS.539400, LOC100133511, HS.153034, miR-129, LOC401629 and LOC641860, and/or an over-expression of one or more or all of LOC442245, FRMD3, IFNE1, MUC19, LOC643345, C15ORF5, MIR155HG, LOC644280 and miR-155 is detected in the patient.

In some embodiments, the patient is administered a therapeutically effective amount of ezatiostat or a salt thereof if an under-expression of a gene of the c-Jun N-terminal kinase gene set is detected. In some embodiments, the patient is administered a therapeutically effective amount of ezatiostat or a salt thereof if an under-expression of all of the c-Jun N-terminal kinase genes is detected.

In some embodiments, the patient is administered with ezatiostat or a salt thereof if an under-expression of at least 5, 10, 15, 20, 25, 30, 35, 40 or 45 of the genes of any one of Tables 1 and 3-5 or the genes of the c-Jun N-terminal kinase genes is detected. In some embodiments, the patient is administered with ezatiostat or a salt thereof if an over-expression of at least 5, 10, 15, 20, 25, 30, 35, 40 or 45 of the genes of Table 2 or 6 is detected. In some embodiments, a patient is administered with ezatiostat or a salt thereof, if an under-expression of multiple genes, selected from Tables 1, 3, 4, and/or 5, for example, at least 10 %, 20 %, 50 % or 75 % of the genes of Tables 1, 3, 4, and/or 5, and/or the c-Jun N-terminal kinase gene set, and/or an over-expression of multiple genes selected from Tables 2 and/or 6, for example, at least 10 %, 20 %, 50 % or 75 % of the genes of Table 2 and/or 6, is detected.

In some embodiments, the genes in Tables 1 and 5 are those having the Entrez Gene IDs or UGIDs described in Table 1A or others known in the art. In some embodiments, the genes in Tables 2 and 6 are those having the Entrez Gene IDs or UGIDs described in Table 2A or others known in the art. In some embodiments, the genes in Table 4 are those having the Entrez Gene IDs or UGIDs described in Table 4A or others known in the art. In some embodiments, the genes are human genes and the patient is a human.

**Table 1A**

| Gene Symbol | Entrez Gene ID | Full Name or Gene Description |
|---|---|---|
| LOC642539 | 642539 | similar to Sucrase-isomaltase, intestinal |
| TNN | 63923, 329278, 424435 | tenascin N |
| LOC651630 | 651630 | similar to nuclear pore membrane protein 121 |
| OR6C6 | 403280 or 283365 | olfactory receptor, family 6, subfamily C, member 6 |
| FAM134A | 79137, 227298, 363252 | family with sequence similarity 134, member A |
| HS.543561 | UGID:1379875 | |
| HS.539400 | UGID:1375714 | |
| C17ORF102 | 400591 | chromosome 17 open reading frame 102 |
| CHAD | 1101, 12643, 29195 | Chondroadherin |
| HS.156574 | UGID:154832 | |
| LOC642228 | 642228 | hypothetical protein LOC642228 |
| LOC650407 | 650407 | hypothetical protein LOC650407 |
| FAM90A5 | 441315 | family with sequence similarity 90, member A5 |
| LOC648408 | 648408 | hypothetical protein LOC648408 |
| RUNDC3B | 154661, 242819, 688590 | RUN domain containing 3B |
| CDRT15L2 | 256223 | CMT1A duplicated region transcript 15-like 2 |
| LOC653579 | 653579 | CD177 molecule pseudogene 1 |
| LOC645781 | 645781 | hypothetical protein LOC645781 |
| CD300E | 342510, 690076, 217306 | CD300e molecule |
| CFL2 | 1073, 12632, 366624 | cofilin 2 (muscle) |
| C8ORF48 | 157773 | chromosome 8 open reading frame 48 |
| LOC651166 | 651166 | hypothetical protein LOC651166 |
| HS.153034 | UGID:154143 | |
| HCRT | 25723,3060 | hypocretin |
| SLC22A9 | 114571 | solute carrier family 22 (organic anion transporter), member 9 |
| DPYSL5 | 56896 | dihydropyrimidinase-like 5 |
| LOC646799 | 646799 | zygote arrest 1-like |
| LOC649125 | 649125 | similar to actin-related protein 2 |
| LOC644059 | 644059 | peptide YY, 3 |
| MIR129-2 | 406918, 723953, 100313984 | microRNA 129-2 |
| OR10A4 | 283297, 768801 | olfactory receptor, family 10, subfamily A, member 4 |
| LOC653536 | 653536 | similar to ST6 beta-galactosamide alpha-2,6-sialyltranferase 2 |
| LOC728255 | 728255 | keratin associated protein 1-4 |
| SNORD72 | 100302529 or 619564 | small nucleolar RNA, C/D box 72 |
| MIR802 | 768219, 791074 | microRNA 802 |
| LOC645839 | 645839 | hypothetical LOC645839 |
| HS.574590 | UGID:1847577 | |
| CPSF1 | 29894, 94230, 366952 | cleavage and polyadenylation specific factor 1, 160kDa |
| LOC401629 | 401629 | non-protein coding RNA 230B |
| FLJ35894 | 283847 | coiled-coil domain containing 79 |
| MIR548E | 100313921, 100315304, 100302231 | microRNA 548e |
| MUC4 | 4585,303887, 140474 | mucin 4, cell surface associated |
| LOC649878 | 649878 | similar to START domain containing 9 |
| LOC642214 | 642214 | similar to SET domain and mariner transposase fusion gene |
| OTOL1 | 131149, 229389, 525044 | otolin 1 |
| LOC641860 | 641860 | hypothetical protein LOC641860 |
| SCNN1G | 6340 or 24768 | sodium channel, nonvoltage-gated 1, gamma |
| LOC340204 | 340204 or 387088 | chromosome 6 open reading frame 127 |
| LOC642561 | 642561 | similar to FXYD domain-containing ion transport regulator 6 |
| LOC100133511 | 100133511 | complement C3-like |
| LOC389730 | 389730 | family with sequence similarity 75, member A6 |
| DAZ3 | 57054 | deleted in azoospermia 3 |
| FAM90A3 | 389611 | family with sequence similarity 90, member A3, pseudogene |
| LRRC8E | 80131,72267, 304203 | leucine rich repeat containing 8 family, member E |
| SLC2A8 | 29988,56017, 85256 | solute carrier family 2 (facilitated glucose transporter), member 8 |
| PAEP | 5047,110645, 280838 | progestagen-associated endometrial protein |
| HS.368690 | UGID:197673 | |
| HS.565863 | UGID:1781352 | |
| KLHDC9 | 126823,68874, 360878 | kelch domain containing 9 |
| LOC652551 | 652551 | hypothetical protein LOC652551 |
| PIGV | 55650,230801, 366478 | phosphatidylinositol glycan anchor biosynthesis, class V |
| TLX1NB | 100038246 | TLX1 neighbor |
| GPC6 | 10082,23888, | glypican 6 |
| HS.545893 | UGID:1382207 | |
| SNX22 | 79856,382083, 300796 | sorting nexin 22 |
| HS.569271 | UGID: 1842258 | |
| LOC654161 | 654161 | similar to AAA-ATPase TOB3 |
| MMP 19 | 4327,58223, 304608 | matrix metallopeptidase 19 |
| ARPM1 | 84517, 76652, 365763 | actin related protein M1 |
| CTGF | 1490 | connective tissue growth factor |
| HS.408168 | UGID:220939 | |
| HS.437395 | UGID:232432 | |
| HS.515967 | UGID:906714 | |
| LOC100133600 | 100133600 | hypothetical protein LOC100133600 |
| LOC645718 | 645718 | hypothetical protein LOC645718 |
| LOC728927 | 728927 | zinc finger protein 736 |
| NRIP3 | 56675, 78593, 361625 | nuclear receptor interacting protein 3 |
| PSMD8 | 5714 | proteasome (prosome, macropain) 26S subunit, non-ATPase, 8 |
| TRIM73 | 375593, 743790 | tripartite motif containing 73 |
| ZBTB7A | 51341, 16969 | zinc finger and BTB domain containing 7A |
| C10ORF116 | 10974 | chromosome 10 open reading frame 116 |
| DEFB127 | 140850, 745453, 100135060 | defensin, beta 127 |
| HS.126108 | UGID:147244 | |
| HS.188979 | UGID:160200 | |
| HS.543520 | UGID:1379834 | |
| HS.553161 | UGID:1506640 | |
| KLC1 | 3831, 16593, 171041 | kinesin light chain 1 |
| KRT36 | 8689 | keratin 36 |
| LOC647928 | 647928 | hypothetical protein LOC647928 |
| PABPC5 | 140886 | poly(A) binding protein, cytoplasmic 5 |
| LOC390530 | 390530 | immunoglobulin heavy variable 1/OR21-1 (non-functional) |

**Table 2A**

| Gene Symbol | Entrez Gene ID | Full Name or Gene Description |
|---|---|---|
| MUC19 | 283463, 239611, 378670, 609857 | mucin 19, oligomeric |
| GLB1L | 79411, 100001628, 74577, 470653 | galactosidase, beta 1-like |
| LOC100129503 | 100129503 | hypothetical LOC100129503 |
| FGF22 | 67112, 27006, 170579 | fibroblast growth factor 22 |
| SRR | 63826, 27364, 303306 | serine racemase |
| IPMK | 253430, 69718, 171458 | inositol polyphosphate multikinase |
| LACE1 | 246269, 215951, 421770 | lactation elevated 1 |
| EIF1AD | 69860, 84285, 615726 | eukaryotic translation initiation factor 1A domain containing |
| FAM114A1 | 92689, 68303, 560911 | family with sequence similarity 114, member A1 |
| LOC100130345 | 100130345 | cadherin-related family member 3-like |
| CLCF1 | 23529, 56708, 365395 | cardiotrophin-like cytokine factor 1 |
| HS.149786 | UGID:153568 | |
| LOC100133639 | 100133639 | hypothetical protein LOC100133639 |
| KIAA1107 | 23285 | KIAA1107 |
| CNP | 12799, 1267, 25275 | 2',3'-cyclic nucleotide 3' phosphodiesterase |
| LOC643345 | 643345 | similar to TBC1 domain family, member 3 |
| HS.550095 | UGID:1436905 | |
| ANKMY2 | 217473, 57037, 420593 | ankyrin repeat and MYND domain containing 2 |
| CCNI2 | 645121, 745286, 708522 | cyclin I family, member 2 |
| LOC730517 | 730517 | similar to MUC19 |
| STRA8 | 20899, 346673, 500079, | stimulated by retinoic acid gene 8 |
| GPX7 | 2882, 67305, 298376 | glutathione peroxidase 7 |
| FRMD3 | 257019, 242506, 298141 | FERM domain containing 3 |
| LOC644280 | 644280 | similar to hCG2041260 |
| TMX3 | 54495, 67988, 553578 | thioredoxin-related transmembrane protein 3 |
| C15ORF5 | 81698 | chromosome 15 open reading frame 5 |
| KHDC1 | 80759, 462818, 100413169 | KH homology domain containing 1 |
| LOC100128088 | 100128088 | matrix metallopeptidase 1-like |
| LOC643402 | 643402 | hypothetical protein LOC643402 |
| LOC100132169 | 100132169 | WASH and IL9R antisense RNA 2 (non-protein coding) |
| MRS2P2 | 729633 | MRS2 magnesium homeostasis factor homolog (*S. cerevisiae)* pseudogene 2 |
| BRCC3 | 79184, 210766, 316794 | BRCA1/BRCA2-containing complex, subunit 3 |
| LOC729242 | 729242 | similar to keratin 17 |
| RBMY3AP | 64593 | RNA binding motif protein, Y-linked, family 3, member A pseudogene |
| OR3A3 | 8392 | olfactory receptor, family 3, subfamily A, member 3 |
| ARL17B | 100506084, 641522 | ADP-ribosylation factor-like 17B |
| LOC440330 | 440330 | hypothetical protein LOC440330 |
| FLJ46109 | 653399 | glutathione S-transferase theta pseudogene 2 |
| PARP8 | 52552, 79668, 294762 | poly (ADP-ribose) polymerase family, member 8 |
| LOC100130982 | 100130982 | hypothetical LOC100130982 |
| FAM83E | 54854, 292913, 73813 | family with sequence similarity 83, member E |
| LOC442245 | 442245 | glutathione S-transferase mu 2 (muscle) pseudogene 1 |
| SYT14 | 255928, 329324, 40544 | synaptotagmin XIV |
| MIR155HG | 114614 | MIR155 host gene (non-protein coding) |
| HS.570965 | UGID:1843952 | |
| TSPAN32 | 27027, 10077, 395087 | tetraspanin 32 |
| IFNE1 | 338376, 230405, 100125969 | interferon, epsilon |
| C14ORF148 | 122945, 780191, 772029 | chromosome 14 open reading frame 148 |
| SLC16A14 | 71781, 151473, 316578 | solute carrier family 16 (monocarboxylic acid transporters), member 14 |
| LOC728297 | 728297 | prostaglandin E2 receptor EP4 subtype-like |
| miR-155 | 777930, 100526376, 100314498, 406947, 387173 | microRNA mir-155 |
| LOC647012 | 647012 | YY1 transcription factor pseudogene |
| LOC649908 | 649908 | hypothetical protein LOC649908 |
| ROPN1 | 54763, 76378, 288053, 527583 | rhophilin associated tail protein 1 |
| AMPD2 | 271, 109674, 362015, 514185, 100584841 | adenosine monophosphate deaminase 2 |
| CNDP1 | 84735, 338403, 614200, 421012 | carnosine dipeptidase 1 (metallopeptidase M20 family) |
| ROCK1 | 6093, 19877, 81762, 373970 | Rho-associated, coiled-coil containing protein kinase 1 |
| ANP32E | 66471, 100172687, 81611, 706251 | acidic (leucine-rich) nuclear phosphoprotein 32 family, member E |
| GAPT | 238875, 202309 | Grb2-binding adaptor, transmembrane |
| SKIL | 6498, 20482, 114208 | SKI-like oncogene |
| CASC1 | 55259, 320662, 465348, 297720 | cancer susceptibility candidate 1 |
| DDO | 8528, 70503, 685325 | D-aspartate oxidase |
| LOC100133435 | 100133435 | similar to melanoma antigen |
| CLEC12A | 160364, 680338, 232413 | C-type lectin domain family 12, member A |
| FLJ41423 | 399886 | uncharacterized LOC399886 |
| GK5 | 256356, 367146, 235533 | glycerol kinase 5 (putative) |
| LOC100131542 | 100131542 | hypothetical LOC100131542 |
| LOC641990 | 641990 | similar to Rho GTPase activating protein 5 isoform b |
| ZNF791 | 163049, 484933 | zinc finger protein 791 |
| EFHC2 | 80258, 74405, 302507 | EF-hand domain (C-terminal) containing 2 |
| LOC730909 | 730909 | uncharacterized LOC730909 |
| PDCD6IP | 10015, 501083, 18571 | programmed cell death 6 interacting protein |
| ZNF193 | 7746, 471917 | zinc finger protein 193 |
| C7ORF46 | 340277, 549482 | chromosome 7 open reading frame 46 |
| CLDN12 | 9069, 64945, 500000 | claudin 12 |
| LOC100129387 | 100129387 | uncharacterized LOC100129387 |
| LOC100132955 | 100132955 | similar to Putative uncharacterized protein C21orf15 |
| LOC647911 | 647911 | hypothetical protein LOC647911 |
| LOC730173 | 730173 | similar to ubiquitin-conjugating enzyme E2C |
| NME2P1 | 283458 | non-metastatic cells 2, protein (NM23B) expressed in, pseudogene 1 |
| NRSN1 | 140767, 22360, 291129 | neurensin 1 |
| PRKCB | 5579, 18751, 25023 | protein kinase C, beta |
| SNAP25 | 6616, 20614, 25012 | synaptosomal-associated protein |
| ABCA2 | 20, 11305, 79248 | ATP-binding cassette, sub-family A (ABC1), member 2 |
| CDC2L5 | 8621, 326776 | cell division cycle 2-like 5 (cholinesterase-related cell division controller) |
| HS.463736 | UGID:678895 | |
| HS.543956 | UGID:1380270 | |
| HS.545655 | UGID:1381969 | |
| KLK6 | 5653, 29245, 19144 | kallikrein-related peptidase 6 |
| LOC100132516 | 100132516 | hypothetical LOC100132516 |
| LOC100133719 | 100133719 | similar to DALR anticodon binding domain containing 3 |
| LOC643717 | 643717 | hypothetical LOC643717 |
| LOC728692 | 728692 | hypothetical LOC728692 |

**Table 4A**

| | Gene Symbol | Entrez Gene ID | Full Name or Gene Description |
|---|---|---|---|
| 4-1. | C1ORF144 | 26099 | chromosome 1 open reading frame 144 |
| 4-2. | IFNA2 | 3440 | interferon, alpha 2 |
| 4-3. | KIF22 | 3835 | kinesin family member 22 |
| 4-4. | ANKRD1 | 27063 | ankyrin repeat domain 1 |
| 4-5. | FLRT1 | 23769 | fibronectin leucine rich transmembrane protein 1 |
| 4-6. | ANXA13 | 312 | annexin A13 |
| 4-7. | PATHWAY:.1NK_UP.v1 | | |
| 4-8. | PLAA | 9373 | phospholipase A2-activating protein |
| 4-9. | BEAN | 146227 | brain expressed, associated with NEDD4, 1 |
| 4-10. | SOX10 | 6663 | SRY (sex determining region Y)-box 10 |
| 4-11. | EIF2C2 | 27161 | eukaryotic translation initiation factor 2C, 2 |
| 4-12. | CYP1A1 | 1543 | cytochrome P450, family 1, subfamily A, polypeptide 1 |
| 4-13. | PLK1 | 5347 | polo-like kinase 1 |
| 4-14. | KRT75 | 9119 | keratin 75 |
| 4-15. | CCL15 | 6359 | chemokine (C-C motif) ligand 15 |
| 4-16. | ARL4A | 10124 | ADP-ribosylation factor-like 4A |
| 4-17. | C10ORF10 | 11067 | chromosome 10 open reading frame 10 |
| 4-18. | TEK | 7010 | TEK tyrosine kinase, endothelial |
| 4-19. | FABP6 | 2172 | fatty acid binding protein 6, ileal |
| 4-20. | TULP1 | 7287 | tubby like protein 1 |
| 4-21. | ABHD2 | 11057 | abhydrolase domain containing 2 |
| 4-22. | C1S | 716 | complement component 1, s subcomponent |
| 4-23. | UBE2C | 11065 | ubiquitin-conjugating enzyme E2C |
| 4-24. | PDGFB | 5155 | platelet-derived growth factor beta polypeptide |
| 4-25. | DYRK3 | 8444 | dual-specificity tyrosine-(Y)-phosphorylation regulated kinase 3 |
| 4-26. | RAB6B | 51560 | RAB6B, member RAS oncogene family |
| 4-27. | JPH3 | 57338 | junctophilin 3 |
| 4-28. | SYT17 | 51760 | synaptotagmin XVII |
| 4-29. | G0S2 | 50486 | G0/G1 switch 2 |
| 4-30. | PIB5PA | 27124 | inositol polyphosphate-5-phosphatase J |
| 4-31. | HIST1H2AM | 8336 | histone cluster 1, H2am |
| 4-32. | KCNN3 | 3782 | potassium intermediate/small conductance calcium-activated channel, subfamily N, member 3 |
| 4-33. | HIST1H2AK | 8330 | histone cluster 1, H2ak |
| 4-34. | MPL | 4352 | myeloproliferative leukemia virus oncogene |
| 4-35. | ASGR1 | 432 | asialoglycoprotein receptor 1 |
| 4-36. | SHC3 | 53358 | SHC (Src homology 2 domain containing) transforming protein 3 |
| 4-37. | RRAD | 6236 | Ras-related associated with diabetes |
| 4-38. | MRAS | 22808 | muscle RAS oncogene homolog |
| 4-39. | CXCL11 | 6373 | chemokine (C-X-C motif) ligand 11 |
| 4-40. | ABP1 | 26 | amiloride binding protein 1 (amine oxidase (copper-containing)) |
| 4-41. | ALOX12B | 242 | arachidonate 12-lipoxygenase, 12R type |
| 4-42. | IL8 | 3576 | interleukin 8 |
| 4-43. | Fll | 2160 | coagulation factor XI |
| 4-44. | PCDH9 | 5101 | protocadherin 9 |
| 4-45. | MMP2 | 4313 | matrix metallopeptidase 2 (gelatinase A, 72kDa gelatinase, 72kDa type IV collagenase) |
| 4-46. | NFKBIL1 | 4795 | nuclear factor of kappa light polypeptide gene enhancer in B-cells inhibitor-like 1 |
| 4-47. | CDKN3 | 1033 | cyclin-dependent kinase inhibitor 3 |
| 4-48. | TACR1 | 6869 | tachykinin receptor 1 |
| 4-49. | ECGF1 | 1890 | thymidine phosphorylase |
| 4-50. | GJA4 | 2701 | gap junction protein, alpha 4, 37kDa |

Genes of the c-Jun N-terminal kinase gene set include JNK1 (four isoforms), JNK2 (four isoforms) and JNK3 (two isoforms).

In some embodiments, the biological sample is bone marrow. In some embodiments, the biological sample is isolated from impurities.

In the methods of the invention, determining the presence of gene expression encompasses detecting presence or absence of gene expression of the gene, as well as determining the level of gene expression of the gene, and is usually performed by testing a biological sample of a patient.

In some embodiments, one or more the steps of determining gene presence and/or expression levels, or correlating data are performed by a computer with appropriate conventional software.

The expression level of a gene may be compared to a baseline gene expression level. A baseline level may be established in several ways. For example, a baseline may be established through creation of a guide that consolidates information on gene expression levels taken from a pool of healthy individuals, patients having MDS who respond to the treatment, or patients who do not respond to the treatment or even from an appropriate cell culture. Further, information on baseline levels of gene expression of a particular gene may be gathered from published sources or a gene database. Thus, baseline could be obtained or established from gene expression level information of similar patient populations. Baseline could also be represented by the gene expression level of a reference standard.

Methods for extraction of biological samples and measuring gene presence and expression are commonly known in the art. Detection can be by any appropriate method, including for example, detecting the quantity of mRNA transcribed from the gene, or the quantity of cDNA produced from the reverse transcription of the mRNA transcribed from the gene, or the quantity of the polypeptide or protein encoded by the gene. Genes may be isolated from cell or tissue samples by conventional methods. For instance, mRNA can be isolated using various lytic enzymes or chemical solutions according to the procedures set forth in Sambrook et al., Molecular Cloning: A Laboratory Manual, 2nd edition (1989), or extracted by nucleic-acid-binding resins following the accompanying instructions provided by manufacturers. The mRNA of the gene contained in the isolated sample can be detected by hybridization (e.g. Northern blot analysis) and/or amplification procedures, such as polymerase chain reaction (PCR), according to methods widely known in the art.

These methods can be performed on a sample by sample basis or modified for high throughput analysis. For example, transcriptional activity of a gene may be assessed by measuring levels of messenger RNA using a gene chip such as the Affymetrix HG-U133-Plus-2 GeneChips. Results from the chip assay can be analyzed using a computer software program known in the art.

Expression level of a gene can also be determined by examining the protein product. Determining the protein level may involve (a) providing a biological sample containing expression product of the gene; and (b) measuring the amount of any immunospecific binding that occurs between an antibody that selectively recognizes and binds to the expression product of the gene in the sample, in which the amount of immunospecific binding indicates the level of the gene expression, or (c) monitoring the binding of a protein that positively or negatively regulates a gene. This information can be compared to a pre-determined baseline and analyzed to identify those patients suitable for the treatment.

A variety of techniques are available in the art for protein or gene analysis, including but not limited to radioimmunoassays, ELISA (enzyme linked immunosorbent assays), "sandwich" immunoassays, immunoradiometric assays, in situ immunoassays (using e.g., colloidal gold, enzyme or radioisotope labels), northern blot analysis, western blot analysis, immunoprecipitation assays, immunofluorescent assays, flow cytometry, immunohistochemistry, confocal microscopy, enzymatic assays, tiling array, DNA microarray and PAGE-SDS.

Also within the scope of this application is a database useful for the identification of patients likely to respond to treatment with ezatiostat or a salt thereof, wherein the database contains gene expression profile data, for example, the gene expression profile of patients who responded to the treatment, and/or the gene expression profile of patients who did not respond to the treatment. Test results of a sample of a patient can be compared against the database using bioinformatic techniques known in the art in order to determine whether the patient is likely to respond to the treatment with ezatiostat or a salt thereof.

In some embodiments of this invention, ezatiostat or a salt thereof, for example, ezatiostat hydrochloride, is administered by a dosing regimen described in U.S. Patent Application Publication US2011/0301102, titled "COMPOSITIONS AND METHODS FOR TREATING MYELODYSPLASTIC SYNDROME," filed May 16, 2011, which is incorporated by reference in its entirety.

Typically, ezatiostat or a salt thereof is administered in a therapeutically effective amount. In some embodiments of this invention, ezatiostat or a salt thereof is administered up to about 3.5 grams per day of ezatiostat hydrochloride, or an equivalent amount (in terms of ezatiostat content) of ezatiostat itself or another salt of ezatiostat. In a preferred embodiment, the dosing of ezatiostat or a salt thereof is a therapeutically effective amount of up to about 1.5 grams administered twice a day (b.i.d.).

In some embodiments, ezatiostat or a salt thereof is administered daily for at least 2 weeks. In some embodiments, ezatiostat or a salt thereof is administered daily for at least 3 weeks.

In one embodiment of this invention, ezatiostat or a salt thereof is administered in 1 gram dosages twice a day for three weeks followed by an interruption of one week where ezatiostat or a salt thereof is not administered. After the interruption, the regimen can be repeated as necessary. This regimen may be referred to as the "three-week regimen."

In another embodiment of this invention, ezatiostat or a salt thereof is administered in 1.5 gram dosages twice a day for two weeks followed by an interruption of one week where ezatiostat or a salt thereof is not administered. After the interruption, the regimen can be repeated as necessary. This regimen may be referred to as the "two-week regimen."

In another embodiment of this invention, the patient is treated continuously with a therapeutically effective amount of ezatiostat or a salt thereof of up to 3 grams per day preferably administered in up to 1.5 gram dosages twice a day. In this embodiment, ezatiostat or a salt thereof can be administered so long as the patient is in need of and can tolerate such treatment. It is contemplated that in this embodiment, the therapeutically effective amount of ezatiostat or a salt thereof may be less or more than that when there is an interruption in the treatment regimen. This regimen may be referred to as the "continuous regimen."

While twice a day administration is preferred, it is contemplated that once a day administration or 3 times a day administration could be employed. In the former case, once a day administration would assist in patient compliance; whereas in the latter case, smaller tablets could be used for those patients who have difficulty swallowing larger tablets. The amount of drug administered would be adjusted so that the total drug administered per day is a therapeutically effective amount.

The treatment with ezatiostat or a salt thereof may involve one or a combination of two or more of the dosing regimens described herein. The following are exemplifying dosing schedules of ezatiostat hydrochloride:
- 1.5 gram ezatiostat hydrochloride administered twice per day for 2 weeks for an aggregate total dosing of 42 grams followed by a week when no ezatiostat or a salt is administered;
- 1 gram ezatiostat hydrochloride administered twice per day for 3 weeks for an aggregate total dosing of 42 grams followed by a week when no ezatiostat or a salt is administered;
- 1 gram ezatiostat hydrochloride administered twice per day continuously until the attending clinician deems it appropriate for the patient to be withdrawn from administration;
- a therapeutically effective amount of up to 3 grams of ezatiostat hydrochloride per day administered in one, two, or three divided doses for 2 weeks followed by a week when no ezatiostat or a salt is administered;
- a therapeutically effective amount of up to 2 grams of ezatiostat hydrochloride per day administered in one, two, or three divided doses for 3 weeks followed by a week when no ezatiostat or a salt is administered; and/or
- a therapeutically effective amount of up to 2 grams of ezatiostat hydrochloride per day administered in one, two, or three divided doses continuously until the attending clinician deems it appropriate for the patient to be withdrawn from administration.

An equivalent amount of ezatiostat or another salt thereof (in terms of ezatiostat content) may replace ezatiostat hydrochloride in the above dosings.

When administration of ezatiostat or a salt thereof is twice a day, it is preferred that the interval between the first and second doses be from about 6 to 14 hours and preferably between about 8 and 14 hours.

In one embodiment, ezatiostat or a salt thereof, e.g., ezatiostat hydrochloride, can be administered intravenously as a lipid formulation such as those described in U.S. Patent No. 7,029,695 which is incorporated by reference in its entirety.

In one embodiment, ezatiostat or a salt thereof can be administered orally. In another embodiment, ezatiostat or a salt thereof can be administered as a tablet formulation. Such a tablet formulation is disclosed in U.S. Patent Application Publication US2011/0300215, filed March 29, 2011, titled "TABLET FORMULATION OF EZATIOSTAT," which is incorporated by reference in its entirety.

In some embodiments, the ezatiostat hydrochloride is an ansolvate or a polymorph thereof as described in U.S. Patent Application Publication US 2011/0301088, filed March 4, 2011, which is incorporated by reference in its entirety. In some embodiments, the ezatiostat is an amorphous form of a pharmaceutically acceptable salt of ezatiostat as described in U.S. Provisional Patent Application No. 61/566,454, filed December 2, 2011, and U.S. Provisional Patent Application No. 61/619,286, filed April 2, 2012, both of which are titled "Amorphous Ezatiostat Ansolvate" and are incorporated by reference in their entirety.

### 3. Assays and Devices

Yet another aspect of the invention is an assay for identifying a patient having a myelodysplastic syndrome to treatment with ezatiostat or a salt thereof, and/or for evaluating the response probability of a patient having a myelodysplastic syndrome to treatment with ezatiostat or a salt thereof, the assay comprising testing a biological sample of the patient, and detecting the presence and/or measuring expression level of a gene selected from Tables 1-6 in the sample. The patient is identified for treatment or considered likely to respond to the treatment if an under-expression of a gene selected from Tables 1 and 3-5, and/or an over-expression of a gene selected from Table 2 or 6 is detected.

Yet another aspect of the invention is an assay for identifying a patient having a myelodysplastic syndrome suitable for treatment with ezatiostat or a salt thereof, wherein the assay comprises testing a biological sample of the patient, detecting the presence and/or measuring expression level of one or more genes selected from Tables 1-6 in the sample, identifying the expression level of said genes, ascertaining the presence of an under-expression of one or more genes selected from Tables 1, 3, 4 and 5, and/or an over-expression of one or more genes selected from Tables 2 and 6, correlating the presence of an under-expression of one or more genes selected from Tables 1, 3, 4 and 5, and/or an over-expression of one or more genes selected from Tables 2 and 6 with the patient's probability of response to the treatment wherein the detection of either an under-expression of one or more genes selected from Tables 1, 3, 4 and 5, and/or an over-expression of one or more genes selected from Tables 2 and 6, indicates that the patient is suitable for treatment with ezatiostat or a salt thereof.

Yet another aspect of the invention is an assay for assaying the response rate of a patient having a myelodysplastic syndrome to treatment with ezatiostat or a salt thereof, the assay comprising means for the detection of an under-expression of a gene selected from Tables 1 and 3-5, and/or an over-expression of a gene selected from Table 2 or 6. The means for the detection includes those known in the art applied to the genes described herein, for example, the methods for determining expression level of a gene, including gene and protein analysis methods, described herein.

Yet another aspect of the invention provides a device for identifying a patient having a myelodysplastic syndrome suitable for treatment with ezatiostat or a salt thereof, said device comprising an output medium that indicates an under-expression of one or more genes selected from Tables 1, 3, 4 and 5, and/or an over-expression of one or more genes selected from Tables 2 and 6.

In some embodiments, the device further comprises a gene chip capable of detecting the presence and/or expression level of one or more genes selected from Tables 1-6.

Yet another aspect of the invention provides a computer-readable medium comprising code which when executed determines whether an under-expression of one or more genes selected from Tables 1, 3, 4 and 5, and/or an over-expression of one or more genes selected from Tables 2 and 6 is present in a sample obtained from a patient having myelodysplastic syndrome. In some embodiments, the code further determines the patient's probability of response to treatment with ezatiostat or a salt thereof, and/or identifies the patient for treatment with ezatiostat or a salt thereof if it is determined that an under-expression of one or more genes selected from Tables 1, 3, 4 and 5, and/or an over-expression of one or more genes selected from Tables 2 and 6 is present.

Yet another aspect of the invention provides a computer-readable medium comprising code which when executed determines a patient's probability of response to treatment with ezatiostat or a salt thereof and/or whether a patient is suitable for treatment with ezatiostat or a salt thereof, taking as an input of whether an under-expression of one or more genes selected from Tables 1, 3, 4 and 5, and/or an over-expression of one or more genes selected from Tables 2 and 6 is present in a sample obtained from the patient.

In some embodiments, the computer-readable medium is in a retrievable form, such as hard drive, computer screen, memory, flash drive, compact disk, cloud server, etc.

Yet another aspect of the invention provides a computer system comprising a processor, a memory, and code which when executed determines whether an under-expression of one or more genes selected from Tables 1, 3, 4 and 5, or an over-expression of one or more genes selected from Tables 2 and 6 is present in a sample obtained from a patient having myelodysplastic syndrome. In some embodiments, the system further determines the patient's probability of response to treatment with ezatiostat or a salt thereof a salt thereof, and/or identifies the patient for treatment with ezatiostat or a salt thereof if it is determined that an under-expression of one or more genes selected from Tables 1, 3, 4 and 5, and/or an over-expression of one or more genes selected from Tables 2 and 6 is present. In some embodiments, the invention provides a computer system comprising a processor, a memory, and code which when executed determines a patient's probability of response to treatment with ezatiostat or a salt thereof and/or whether a patient is suitable for treatment with ezatiostat or a salt thereof taking as an input of whether an under-expression of one or more genes selected from Tables 1, 3, 4 and 5, and/or an over-expression of one or more genes selected from Tables 2 and 6 is present in a sample obtained from the patient. In some embodiments, the computer system provides the determination in a retrievable form, such as information stored in a memory, flash drive, compact disk, cloud server, printed documents, etc.

In some embodiments of the assay and device aspects, the one or more genes are those described herein.

In another aspect, the invention provides a method of using the assays, devices or systems for determining a patient's probability of response to treatment with ezatiostat or a salt thereof and/or identifies a patient for treatment with ezatiostat or a salt thereof.

### Example

The present invention is further defined by reference to the following example.

### Gene Expression Studies For Identifying Myelodysplastic Syndrome Patients Likely To Respond To Therapy With Ezatiostat Hydrochloride

A bedside to bench approach was used to generate a preliminary profile that characterizes patients who responded to treatment with ezatiostat hydrochloride. Based on multilineage responses in low risk and intermediate-1 risk (low-Intl) MDS patients in a phase II study of oral ezatiostat hydrochloride, a multi-institutional phase II study was conducted in low-Intl MDS patients. All patients had low- or intermediate-1-risk MDS as determined by the International Prognostic Scoring System (IPSS) and had not received growth factors for 4 weeks prior to study enrollment. Response was evaluated by the International Working Group (IWG 2006) criteria (Greenberg P, et al., International scoring system for evaluating prognosis in myelodysplastic syndromes. Blood 1997;89(6):2079-88; and Cheson BD, et al., Clinical application and proposal for modification of the International Working Group (IWG) response criteria in myelodysplasia. Blood 2006; 108:419-25). Pre-therapy bone marrow mononuclear cells of patients treated with ezatiostat hydrochloride were stored in Trizol® (Invitrogen Corp., Carlsbad, CA) at -80 °C. The experiment was conducted with Institutional Review Board (IRB) approval.

### Microarrays

Total RNA was purified from 5-10 x 10⁶ mononuclear cells using Trizol® and analyzed for gene expression on the Illumina HT12v4 whole genome array according to the manufacturer's protocol. RNA isolated from the marrow mononuclear cells was available on 9 responders and 21 non-responders. The nine responders included one with a baseline single erythroid cytopenia, one with a single platelet cytopenia, one with erythroid-neutrophil cytopenias, two with erythroid-platelet cytopenias, two with neutrophil-platelet cytopenias and two with trilineage cytopenia. The non-responders included 11 patients with a single erythroid cytopenia, one with single platelet cytopenia, one with single neutrophil cytopenia, two with erythroid-platelet cytopenias, two with erythroid-neutrophil cytopenias, and one with trilineage cytopenias. There were 18 patients with refractory anemia (RA); eight with RA with ringed sideroblasts (RARS); three with RA with excess blasts, type 1 (RAEB-1); and one with RAEB-2. Patient samples had similar representation in both the responder and the non-responder groups.

Five responders and 13 non-responders were randomly chosen to create a training set with the intent to later use the remaining samples for model testing. The top 100 differentially expressed genes were identified using a sensitive metric based on the normalized and rescaled mutual information. Single-sample Gene Set Enrichment Analysis was also performed to find the most salient differences in terms ofpathways and biological processes between responders and non-responders.

### Gene Marker Analysis

The selection of genes associated with the responders (R) vs. non-responders (NR) phenotype was obtained using a normalized and rescaled mutual information score (NMI). This quantity was obtained using a kernel-density-based estimate of the joint probability distribution and the mutual information between the phenotype and each gene profile. The resulting mutual information (Cover T and Thomas J, Elements of Information Theory, 2nd. Ed. Wiley Series in Telecommunications and Signal Processing (2006)) was then normalized by the joint-entropy in order to provide a more universal metric (Li M, et al., The similarity metric. IEEE Trans. on Inf. Theory 2004; 50(12): 3250-3264), rescaled to the interval [0, 1], and assigned a "directionality" sign defined according to the sign of the Pearson correlation between the phenotype and the gene profile. A perfect gene-phenotype match (anti-match) using this NMI score corresponds to a +1(-1) value, and a random match attains approximately 0. The significance of a given NMI score is typically estimated by a permutation test where the values of the phenotype are randomly permutated many times, and a nominal p-value is computed according to how many times the matching scores of the random permutations are more extreme than the actual score. A permutation test was not performed. 100 genes with the highest (50) and lowest (50) NMI scores were analyzed.

### Gene Set/Pathway Analysis

Independently of the gene markers analysis described above, the gene profiles were projected into the space of pathways using a single-sample Gene Set Enrichment Analysis (ssGSEA) (Barbie DA, et al. Systematic RNA interference reveals that oncogenic KRAS-driven cancers require TBK1. Nature 2009;462(7269): 108-12; Subramanian A, et al. Gene set enrichment analysis: a knowledge-based approach for interpreting genome-wide expression profiles. Proc Natl Acad Sci USA. 2005;102(43):15545-50; Jagani Z, et al. Loss of the tumor suppressor Snf5 leads to aberrant activation of the Hedgehog-Gli pathway. Nat Med. 2010;16:1429-1433; Wolfer A, et al. MYC regulation of a "poor-prognosis" metastatic cancer cell state. Proc Natl Acad Sci USA. 2010;107(8):3698-703; Cho YJ, et al. Integrative Genomic Analysis of Medulloblastoma Identifies a Molecular Subgroup That Drives Poor Clinical Outcome. J Clin Oncol. 2011;29(11):1424-30; Tamayo P, et al. Predicting relapse in patients with medulloblastoma by integrating evidence from clinical and genomic features. J. Clin Oncol. 2011; 29(11):1415-23.)

The gene-expression values were first rank-normalized and sorted independently, sample per sample. Then a per-gene enrichment score for each gene set/pathway was computed based on the total weighted difference between the empirical cumulative distribution functions (CDF) of: i) the genes in the gene set vs. ii) the genes not in the set. This procedure is similar to the computation of standard Gene Set Enrichment Analysis (Subramanian A, et al. Proc Natl Acad Sci U S A. 2005;102(43):15545-50), but it is based on absolute rather than differential expression and the total difference rather than the maximum deviation from zero of the CDF.

The selection of gene sets/pathways more associated with the responders vs. non-responders phenotype was obtained using a normalized and rescaled mutual information score (NMI) as was done with the gene profiles (see above). The sources of gene sets/pathways were: i) the C2 sub-collection of curated and functional gene sets from the Molecular Signatures Database (MSigDB) release 2.5 (www.broadinstitute.org/msigdb); ii) an internal database of signatures of oncogene activation containing over 300 gene sets defined from data generated in our laboratory, from GEO datasets, and from the biomedical literature; and iii) gene sets representing hematopoietic cell populations, Novershtern N, et al. Densely interconnected transcriptional circuits control cell states in human hematopoiesis. Cell 2011; 144(2):296-309. A total of 2,776 gene sets were considered. The selection analysis was restricted to the 60 gene sets/pathways with the 30 highest and 30 lowest NMI scores.

The top 100 marker genes (50 under-expressed and 50 over-expressed in the responders, Tables 1 and 2, respectively) were identified using a sensitive metric based on the normalized mutual information (NMI). Most notably, there are two microRNA (miR) genes that are differentially expressed. Responders under-express miR-129 and over-express miR-155. miRNAs are small non-coding RNAs of 18-25 nucleotides that bind the 3' UTR of mRNA, resulting in suppressed translation or mRNA degradation.

Single-sample Gene Set Enrichment Analysis was performed to find the most salient differences in terms of pathways and biological processes between responders and non-responders. Most notably, three pathways, mTOR, JAK2 and JNK, were all found to be under-expressed in the responders (Table 3).

Lastly, and most striking, was the finding that the JNK/JUN pathway is also under-expressed in responding patients. This gene set, as defined by the GEO dataset GDS2081, was derived from expression studies in primary cultured human epidermal keratinocytes, with activated JNK/JUN exposed to the JNK inhibitor drug SP600125 and analyzed on Affymetrix HGU95Av2 arrays (Gazel A, et al. Inhibition of JNK promotes differentiation of epidermal keratinocytes, J Biol Chem. 2006; 281(29):20530-41). A heatmap of responders/non-responders was derived from the combined enrichment score of the top/bottom 200 genes, of which the top expressing genes are shown in Table 4. Most notably, the gene-set profile of the JNK-inhibited keratinocytes was found to be highly similar to the gene-set profile of patients who respond to ezatiostat.

## Claims

1. Ezatiostat or a salt thereof for use in treating a myelodysplastic syndrome in a patient, wherein the patient is detected to have an under-expression of a gene selected from Table 1, 3, 4 or 5, and/or an over-expression of a gene selected from Table 2 or 6.

2. The ezatiostat or a salt thereof of claim 1, wherein the patient is detected to have an under-expression of one or more genes of the c-Jun N-terminal kinase gene set, such as all of the c-Jun N-terminal kinase genes.

3. The ezatiostat or a salt thereof of claim 1, wherein the patient is administered a therapeutically effective amount of ezatiostat or a salt thereof if an under-expression of one or more genes of the mTOR, JAK2 or JNK pathway is detected in the patient.

4. A method of identifying a patient having a myelodysplastic syndrome for treatment with ezatiostat or a salt thereof, said method comprising the *in vitro* testing of a biological sample of the patient to detect the presence and/or measure expression level of a gene selected from Tables 1-6, wherein the patient is identified for the treatment if an under-expression of a gene selected from Table 1, 3, 4 or 5 is detected, and/or an over-expression of a gene selected from Table 2 or 6 is detected.

5. The ezatiostat or a salt thereof of claim 1 or the method of claim 4, wherein the under-expressed gene is miR-129 or the over-expressed gene is miR-155.

6. The method of claim 4, wherein the patient is identified for the treatment if an under-expression of one or more genes of the mTOR, JAK2 or JNK pathway is detected.

7. A method for evaluating the response probability of a patient having a myelodysplastic syndrome to treatment with ezatiostat or a salt thereof, said method comprising the *in vitro* testing of a biological sample of the patient, and detecting the presence and/or measuring expression level of a gene selected from Tables 1-6, wherein the patient is likely to respond to treatment if an under-expression of a gene selected from Table 1, 3, 4 or 5 is detected, and/or an over-expression of a gene selected from Table 2 or 6 is detected.

8. The method of claim 4 or claim 7, wherein an under-expression of a gene selected from Table 1, 3 or 4 is detected.

9. The method of claim 4 or claim 7, wherein an over-expression of a gene selected from Table 2 is detected.

10. The method of claim 7, wherein the gene is miR-129 or miR-155.

11. The method of claim 7, wherein detection of an under-expression of miR-129, and/or detection of an over-expression of miR-155 is indicative that the patient is likely to respond to the treatment.

12. The method of claim 4 or claim 7, wherein the gene is one or more genes of the c-Jun N-terminal kinase gene set.

13. The method of claim 7, wherein detection of an under-expression of one or more genes of the mTOR, JAK2 or JNK pathway is indicative that the patient is likely to respond to the treatment.

14. The method of any one of claims 4-13, wherein the biological sample is bone marrow.

15. An assay for identifying a patient suffering from a myelodysplastic syndrome for whom there is an enhanced probability of response to treatment with ezatiostat or a salt thereof, wherein the assay comprises
detecting the presence and/or measuring expression level of a gene selected from Tables 1-6 in the sample,
identifying the expression level of said genes,
ascertaining the presence of an under-expression of one or more genes selected from Tables 1, 3, 4 and 5, and/or an over-expression of one or more genes selected from Tables 2 and 6,
correlating the presence of an under-expression of one or more genes selected from Tables 1, 3, 4 and 5, and/or an over-expression of one or more genes selected from Tables 2 and 6 with the patient's probability of response to the treatment wherein the detection of either an under-expression of one or more genes selected from Tables 1, 3, 4 and 5, or an over-expression of one or more genes selected from Tables 2 and 6, indicates that the patient has an enhanced likelihood of response to treatment with ezatiostat or a salt thereof.

16. An assay for assaying the response rate of a patient suffering a myelodysplastic syndrome to treatment with ezatiostat or a salt thereof, the assay comprising means for the detection of an under-expression of a gene selected from Tables 1 and 3-5, and/or an over-expression of a gene selected from Table 2 or 6.

17. A device for identifying a patient suffering from a myelodysplastic syndrome for whom there is an enhanced probability of response to treatment with ezatiostat or a salt thereof, said device comprising an output medium that indicates whether there is an under-expression of one or more genes selected from Tables 1, 3, 4 and 5, and/or an over-expression of one or more genes selected from Tables 2 and 6 in the patient.
